(19) Europäisches Patentamt
European Patent Office
Office européen des brevets

(11) Veröffentlichungsnummer: **0 026 846**
**B1**

(12) # EUROPÄISCHE PATENTSCHRIFT

(45) Veröffentlichungstag der Patentschrift: **23.06.82**

(21) Anmeldenummer: **80105270.5**

(22) Anmeldetag: **04.09.80**

(51) Int. Cl.³: **C 07 D 495/22,** C 07 D 495/18, H 01 L 29/28, H 01 B 1/00 // (C07D495/22, 341/00, 339/00, 339/00, 339/00, 339/00),(C07D495/18, 339/00, 339/00, 339/00)

(54) **Cyclisch substituierte Fulvalenophane, ihre Herstellung und Verwendung.**

(30) Priorität: **14.09.79 DE 2937225**

(43) Veröffentlichungstag der Anmeldung: **15.04.81 Patentblatt 81/15**

(45) Bekanntmachung des Hinweises auf die Patenterteilung: **23.06.82 Patentblatt 82/25**

(84) Benannte Vertragsstaaten: **DE FR GB NL**

(56) Entgegenhaltungen:

**De-A-2 525 190**
**DE-A-2 739 584**
**US-A-3 941 809**

(73) Patentinhaber: **BASF Aktiengesellschaft, Carl-Bosch-Strasse 38, D-6700 Ludwigshafen (DE)**

(72) Erfinder: **Staab, Heinz, Dr., Schloss-Wolfsbrunnenweg 43, D-6900 Heidelberg (DE)**
Erfinder: **Ippen, Joachim, Dr., Morgengraben 14, D-5000 Köln 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 026 846 B1

BUNDESDRUCKEREI BERLIN

## Cyclisch substituierte Fulvalenophane, ihre Herstellung und Verwendung

Diese Erfindung betrifft neue cyclische substituierte Fulvalenophane, ein Verfahren zu ihrer Herstellung und ihre Verwendung.

In der US-A-3 941 809, DE-A-2 525 190 und DE-A-2 739 584 werden eine Reihe von schwefel-, selen- oder tellurhaltigen Heterofulvalenen mit leitenden Eigenschaften beschrieben, gegenüber denen die untenstehend definierten Fulvalenophane strukturell sehr unterschiedlich sind und überraschenderweise überlegene Leitfähigkeitseigenschaften aufweisen.

Die neuen erfindungsgemäßen Fulvalenophane sind interessante organische Leiter oder Halbleiter oder Vorprodukte zu solchen Stoffen. Sie haben die Formel

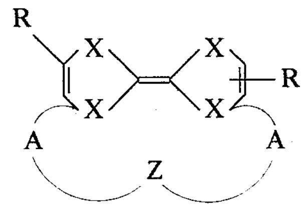

(I)

in der X S, Se oder Ze, R H oder niedere Alkyl, mit 1—5 C-Atomen A einen $-(CH_2)_n$-Rest, in dem n für 2, 3, 4 oder 5 steht und ein oder mehrere $-CH_2$-Glieder durch die Gruppen $-S-$, $-SO_2-$, $-O-$, $-NH-$, $-NR^1-$, $-CO-$, $-O-CO-$, $-O-CO-O-$, $-NR^1-CO-$, in denen $R^1$ für einen Alkylrest mit 1 bis 5 C-Atomen steht, ersetzt sein können und Z einen Rest der Formel

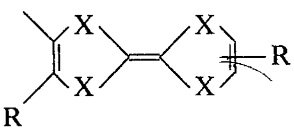

einen p-Phenylrest, der durch Halogenatome, Nitrogruppen oder Cyangruppen substituiert sein kann, oder einen Rest der Formel

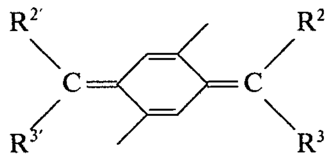

in dem die Reste $R^2$ und $R^3$ Halogenatome, Carbonsäureestergruppen oder Cyangruppen bedeuten und $R^3$ außerdem für ein Wasserstoffatom stehen kann, und der Chinodimethanring durch Halogenatome, Nitrogruppen oder Cyangruppen substituiert sein kann, bedeuten. Der niedere Alkylrest R steht für einen Alkylrest mit 1 bis 5 C-Atomen, vorzugsweise für Methyl.

Von technischem Interesse sind z. B. die Tetrathiofulvalene der Formel

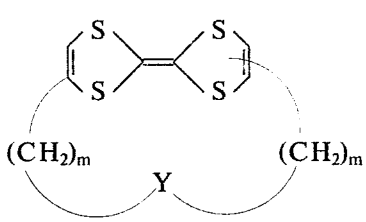

(II)

in der m die Zahl 2, 3, 4 oder 5 und Y einen der Reste

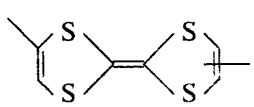

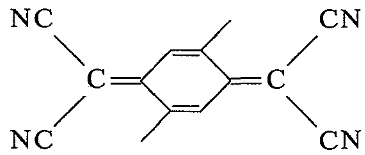

oder einen p-Phenylenrest, der Cyanogruppen, Nitrogruppen oder Halogenatome enthalten kann, bedeuten.

Beispielsweise seien die Tetrathiofulvalenophane der Formel

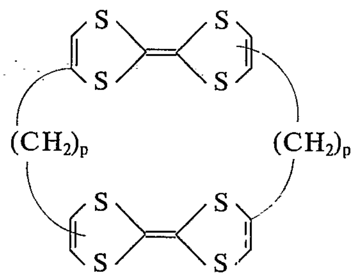

(III)

in der p für 2, 3 oder 4 steht oder der Formel

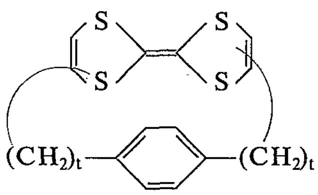

(IV)

in der t für 3, 4 oder 5 steht, genannt.

Man stellt die neuen cyclisch substituierten Fulvalenophane unter Verwendung an sich bekannter Verfahren dadurch her, daß man eine Verbindung der Formel

$$\text{Hal}-(\text{R})\text{HC}-\text{OC}-\text{E}-\text{CO}-\text{CH}(\text{R})-\text{Hal} \qquad (\text{V})$$

in der Hal Chlor oder Brom und E einen $-(CH_2)_n-$Rest oder einen Rest der Formel $-A-Z-A-$ bedeuten

a) durch Umsetzung mit Verbindungen der Formel

$$\text{KX}-\text{CX}-\text{OCH}(\text{CH}_3)_2 \qquad (\text{VI})$$

in eine Verbindung der Formel

$$[(\text{CH}_3)_2\text{CH}-\text{O}-\text{CX}-\text{X}-\text{CH}(\text{R})-\text{CO}]_2\text{E} \qquad (\text{VII})$$

überführt,

b) die Verbindung der Formel VII durch Behandlung mit Säuren zu einer Verbindung der Formel

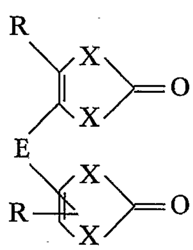

(VIII)

cyclisiert,

c) die Verbindung der Formel VIII durch Behandlung mit Phosphor-(V)-sulfid zu der Verbindung der Formel

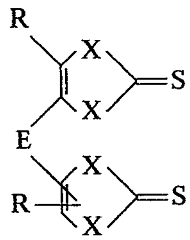

(IX)

umsetzt,

d) die Verbindung der Formel IX durch Methylierung und Reduktion in die Verbindung der Formel

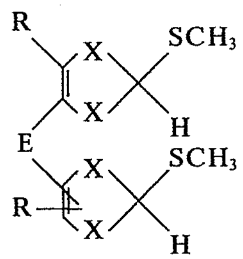

(X)

überführt,

e) die Verbindung der Formel X durch Behandlung mit Fluorborsäure in das Tetrafluoborat der Formel

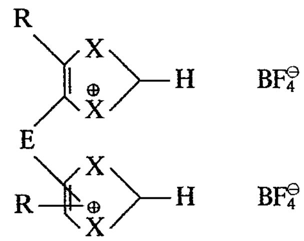

(XI)

überführt und

f) das Fluoborat der Formel XI mit tertiären Aminen behandelt.

Die Ausgangsstoffe der Formel V sind z. B. aus den Säurechloriden der Formel Cl—OC—E—CO—Cl und Diazomethan und nachfolgende Behandlung der Bisdiazoverbindung mit Halogenwasserstoff erhältlich.

Die Bis(halogenketone) der Formel V werden gemäß Stufe (a) mit Verbindungen der Formel VI, z. B. Kalium—O—isopropyldithiocarbonat ($KS-CS-O-CH(CH_3)_2$), in einem Lösungsmittel, wie Aceton bei Temperaturen von 20 bis 55° C umgesetzt.

Die Cyclisierung der Verbindung der Formel VII gemäß Stufe (b) nimmt man mit Säuren, z. B. mit konzentrierter Schwefelsäure oder mit 70%iger Perchlorsäure bei 20 bis 50° C vor.

Die Verbindung der Formel VIII setzt man gemäß Stufe (c) zweckmäßig in Xylol bei Temperaturen von 120 bis 140° C mit dem Phosphor(V)sulfid um.

Man methyliert die Verbindung der Formel IX gemäß Stufe (d) zweckmäßig bei 50 bis 80° C in Nitromethan mit Methyljodid und reduziert das dabei erhältliche Reaktionsprodukt, vorteilhaft ohne es zu isolieren, z. B. mit Natriumborhydrid. Diese Reduktion, bei der man die Verbindung der Formel X erhält, führt man z. B. bei Temperaturen von —78 bis 50° C in einem Gemisch aus Tetrahydrofuran und Methanol durch.

Das Fluoborat der Formel XI wird aus der Verbindung der Formel X gemäß Stufe (e) durch Umsetzung mit Fluorborsäure, z. B. in Acetanhydrid bei Temperaturen von 0 bis 30° C hergestellt. Schließlich cyclisiert man gemäß Stufe (f), indem man das Fluoborat der Formel XI mit tertiären Aminen behandelt. Diese Reaktion wird z. B. durchgeführt, indem man in eine Lösung des Fluoborates in Acetonitril bei Temperaturen von 0 bis 40° C eine Lösung von Triethylamin in Acetonitril eintropft.

Die erfindungsgemäßen Fulvalenophane sind organische Leiter, Photo- oder Halbleiter oder Vorprodukte solcher Stoffe.

Nach einer weiteren Ausführungsform der Erfindung kann man die erfindungsgemäßen Fulvalenophane auch für die Herstellung weiterer organischer Leiter, Photo- oder Halbleiter verwenden, indem man sie als solche einsetzt oder mit Verbindungen der Formel

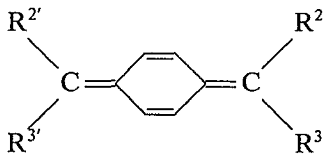

(XIII)

in der die Reste $R^2$ und $R^3$ Halogenatome, Carbonsäureestergruppen oder Cyangruppen bedeuten und $R^3$ außerdem für ein Wasserstoffatom stehen kann, und der Chinodimethanring durch Halogenatome, Nitrogruppen oder Cyanogruppen substituiert sein kann, oder mit substituierten Benzolen, wie Tetracyanobenzol, behandelt.

Bei dieser Umsetzung, die man z. B. bei Temperaturen von 0 bis 50° C in Acetonitril/Schwefelkohlenstoff durchführt, werden Komplexverbindungen erhalten, die z. B. auf 1 Mol Fulvalenophan 1 bis 4 Mol der Chinodimethanverbindun der Formel XIII oder der Benzolverbindung enthalten.

## Beispiel 1

### Herstellung von [2,2]-Tetrathiafulvalenophan der Formel III

#### 1,6-Bisdiazo-2,5-dioxohexan

Zu 600 ml (0,5 Mol) einer Diazomethan-Lösung wird unter Rühren und Eiskühlung eine Lösung von 9,7 g (62,5 mMol) Bernsteinsäuredichlorid in 100 ml Äther langsam eingetropft. Man läßt noch 3 Stunden unter Eiskühlung nachrühren und dann bei Raumtemperatur über Nacht stehen. Man gießt die Lösung ab und bläst das überschüssige Diazomethan sowie ein Teil des Äthers ab. Man gießt die Lösung von einem braunen Rückstand ab und setzt die Lösung, nachdem ein Volumen von ca. 300 ml erreicht ist, ohne weitere Reinigung für die nächste Stufe ein.

#### 1,6-Dichlor-2,5-dioxohexan

Eine Lösung von ca. 62 mMol 1,6-Bisdiazo-2,5-dioxohexan in Äther wird langsam unter Rühren und Eiskühlung in 300 ml einer gesättigten Äther/Chlorwasserstoffgas-Lösung getropft. Es ist eine Gasentwicklung zu beobachten und es tritt eine Trübung auf. Es wird noch 3 Stunden bei Raumtemperatur nachgerührt und dann über Nacht stehen gelassen. Die Lösung wird am Rotationsverdampfer eingeengt, wobei eine kristalline Substanz und etwas Öl zurückbleibt. Es werden 20 ml Äthanol zugegeben und das weiße kristalline Produkt abgesaugt. Durch Umkristallisation aus Cyclohexan erhält man farblose Plättchen, die bei 87—88° C schmelzen. Die Ausbeute beträgt 2,6 g 1,6-Dichlor-2,5-dioxohexan (23%).

#### 2,15-Dimethyl-3,14-dioxa-7,10-dioxo-5,12-dithia-4,13-dithioxohexadecan (s. Formel VII, mit R=H; X=S und E= $-(CH_2)_2-$)

In 50 ml Aceton werden 1,7 g (9,28 mMol) 1,6-Dichlor-2,5-dioxohexan gelöst und 3,2 g (18,5 mMol) Kaliumisopropylxanthogenat bei Raumtemperatur zugegeben. Es fällt sofort ein farbloser breiiger Niederschlag aus. Bei Raumtemperatur wird noch 3 Stunden nachgerührt. Man gießt in 200 ml Wasser und extrahiert mit Methylenchlorid. Die Methylenchloridphase wird getrocknet und eingeengt. Man erhält leicht braune Kristalle, die aus Tetrachlorkohlenstoff umkristallisiert werden. Die Ausbeute beträgt 3 g 2,15-Dimethyl-3,14-dioxa-7,10-dioxo-5,12-dithia-4,13-dithioxohexadecan (84%). Schmelzpunkt: 82—83° C.

#### 1,2-Bis(isodithionyl)-äthan (s. Formel VIII mit R=H, X=S und E= $-(CH_2)_2-$)

In 20 ml konzentrierter Schwefelsäure werden unter Eiskühlung langsam in kleinen Portionen 3 g (7,8 mMol) 2,15-Dimethyl-3,14-dioxa-7,10-dioxo-5,12-dithia-4,13-dithioxohexadecan eingetragen. Man läßt 2 Stunden bei Raumtemperatur nachrühren und gießt dann die dunkelbraune Lösung in Eiswasser ein. Die farblose Suspension wird mit Äther extrahiert. Die Ätherphase wird mit Wasser gewaschen, über Natriumsulfat getrocknet und eingeengt. Es resultieren weiße Kristalle, die über eine Kieselgelsäule mit dem Laufmittel Methylenchlorid gegeben werden. Nach der Umkristallisation aus Toluol erhält man 1,2-Bis-(isodithionyl)-äthan in Form langer weißer Nadeln mit dem Schmelzpunkt von 135—136° C. Die Ausbeute beträgt 1,8 g (87%).

1,2-Bis(isotrithionyl)-äthan (s. Formel IX mit R = H, X = S und E = $-(CH_2)_2-$)

In 50 ml Xylol werden 1,8 g (6,8 mMol) 1,2-Bis(isodithionyl)-äthan und 1,5 g Phosphorpentasulfid 6 Stunden am Rückfluß gekocht. Nach dem Abkühlen wird das Lösungsmittel abfiltriert und der Rückstand mehrmals mit Toluol ausgekocht. Die vereinigten Lösungsmittelextrakte werden eingeengt, wobei eine orange kristalline Substanz zurückbleibt. Das Produkt wird 3mal aus Toluol umkristallisiert. Die Ausbeute an 1,2-Bis(isotrithionyl)-äthan beträgt 1,6 g (80%). Der Schmelzpunkt ist 172—174° C.

1,2-Bis(2'-methylthio-1',3'-dithiol-4'-yl)-äthan (s. Formel X mit E = $-(CH_2)_2-$, R = H, X = S)

Eine Suspension von 205,8 mg (0,7 mMol) des 1,2-Bis(isotrithionyl)-äthans in 3 ml Nitromethan und 2 ml Methyljodid wird 2,5 Stunden bei 55° C dann über Nacht bei Raumtemperatur verrührt. Durch eine Dünnschichtchromatographie wird die Vollständigkeit der Reaktion überprüft. Das Lösungsmittel wird mit Argon abgeblasen und der gelbe Rückstand an der Ölpumpe getrocknet. Das so erhaltene Bis-sulfoniumjodid wird ohne weitere Reinigung wie folgt hydriert (Rohausbeute 403 mg (100%)):

In 4 ml absolutem Tetrahydrofuran und 4 ml absolutem Methanol werden 403 mg (0,7 mMol) Bis-sulfoniumjodid suspendiert und auf −78° C abgekühlt. In kleinen Portionen werden 262 mg (7 mMol) Natriumborhydrid zugegeben und 3 Stunden bei −78° C nachgerührt. Innerhalb 2,5 Stunden läßt man die Temperatur von −78° C auf +10° C ansteigen, wobei das Reaktionsgemisch stark aufschäumt. Die gelbe Suspension wird in 100 ml Äther gegossen und mit 100 ml Wasser gewaschen. Die Wasserphase wird mit 100 ml Äther und die beiden Ätherphasen zweimal mit Wasser und einmal mit gesättigter Natriumchloridlösung gewaschen. Die Ätherphase wird über Natriumsulfat getrocknet und eingeengt. Man erhält ein gelbes Öl, welches unter einer Argonatmosphäre mit Methylenchlorid und Tetrachlorkohlenstoff im Verhältnis 2 : 1 als Laufmittel über eine Kieselgelsäule (Länge 30 cm, Ø 1 cm) filtriert wird. Das Filtrat wird eingeengt und das Produkt der Formel X bleibt in Form eines gelben empfindlichen Öles zurück. Die Ausbeute liegt zwischen 27 und 40%.

Bis-dithioliumion (s. Formel XI mit R = H, E = $-(CH_2)_2-$ und X = S)

Unter Stickstoff werden 300 mg (0,92 mMol) 1,2-Bis (2'-methylthio-1',3'-dithiol-4'-yl)-äthan in 6,5 ml Essigsäureanhydrid vorgelegt und auf 0° C abgekühlt. Dann addiert man tropfenweise innerhalb von 15 Minuten 0,9 ml Fluorborsäure. Man erhält eine braune Lösung, die noch 40 Minuten bei 0° C nachgerührt wird. In dieser Zeit fällt etwas hellbrauner Niederschlag aus. Man gibt 50 ml absoluten Äther hinzu, wobei mehr brauner Niederschlag ausfällt. Nach kurzem Durchrühren wird der Äther abpipettiert und der Niederschlag noch 5mal auf die gleiche Art und Weise gewaschen. Der hellbraune Niederschlag wird an der Ölpumpe getrocknet und dann ohne weitere Reinigung in der nächsten Stufe eingesetzt.

[2,2]-Tetrathiafulvalenophan

Unter einer Argonatmosphäre gibt man bei Raumtemperatur tropfenweise eine Lösung aus (0,92 mMol) Bis-dithioliumion in 30 ml absolutem Acetonitril zu einer Lösung von 3 ml Triäthylamin in 50 ml absolutem Acetonitril. Die Zugabe dauert 45 Minuten. Es wird noch 1 Stunde bei Raumtemperatur gerührt und unter Argon filtriert. Die orange Lösung wird am Rotationsverdampfer eingeengt und der Rückstand an der Ölpumpe getrocknet. Durch fraktionierte Umkristallisation aus Schwefelkohlenstoff erhält man in einer Ausbeute von 10 bis 15% [2,2]-Tetrathiafulvalenophan in Form gelber Kristalle, die sich bei 210° C schwarz färben und bis 310° C nicht schmelzen.

Beispiel 2

Herstellung von [3,3]-Tetrathiafulvalenophan der Formel III

1,7-Bisdiazo-2,6-dioxoheptan

In einem Erlenmeyerkolben werden 600 ml (0,5 Mol) einer Diazomethan-Lösung vorgelegt. Dann wird eine Lösung von 16,9 g (0,1 Mol) Glutarsäuredichlorid in 200 ml Ether langsam unter Rühren und Eiskühlung eingetropft. Man läßt 3 Stunden unter Eiskühlung nachrühren und dann bei Raumtemperatur über Nacht stehen. Das überschüssige Diazomethan sowie ein Teil des Ethers werden mit Preßluft hinter einer Schutzscheibe in einem gut ziehenden Abzug abgeblasen. Nachdem ein Lösungsvolumen von ca. 300 ml erreicht ist, wird das Produkt ohne Reinigung in der nächsten Stufe eingesetzt.

1,7-Dichlor-2,6-dioxoheptan

Zu 300 ml einer mit gasförmigem Chlorwasserstoff gesättigten Ether-Lösung werden unter Rühren und Eiskühlung eine Lösung von ca. 100 mMol des 1,7-Bisdiazo-2,6-dioxoheptans in 300 ml Ether getropft. Es tritt eine Trübung auf und Gasentwicklung ist zu beobachten. Man läßt noch 2 Stunden bei Raumtemperatur nachrühren und dann über Nacht stehen. Das farblose ausgefallene Produkt wird abgesaugt und mit Ether nachgewaschen. Der Schmelzpunkt des in wechselnden Mengen anfallenden Produktes beträgt 74—75° C.

Die Etherphase wird vorsichtig mit Wasser versetzt und mit Natriumhydrogencarbonat auf pH = 6 eingestellt. Die Etherphase wird abgetrennt und mit Natriumsulfat getrocknet. Nach dem Einengen bleibt ein farbloses Öl zurück, welches nach dem Abkühlen auf 0° C durchkristallisiert. Durch Umkristallisieren aus Cyclohexan erhält man 1,7-Dichlor-2,6-dioxoheptan in Form farbloser Nadeln, die bei 74—75° C schmelzen. Die Gesamtausbeute beträgt 11 g (43%).

2,16-Dimethyl-3,15-dioxa-7,11-dioxo-5,13-dithia-4,14-dithioxoheptadecan (s. Formel VII mit R = H, X = S und E = $-(CH_2)_3-$)

In 75 ml Aceton werden 3,8 g (14 mMol) 1,7-Dichlor-2,6-dioxoheptan vorgelegt und 4,8 g (28 mMol) Kaliumisopropylxanthogenat bei Raumtemperatur zugegeben. Es fällt sofort ein farbloser breiiger Niederschlag aus. Man läßt noch 2 Stunden bei Raumtemperatur nachrühren, gießt dann in 200 ml Wasser und extrahiert mit Ether. Die Etherphase wird getrocknet und eingeengt. Man erhält farblose Kristalle, die aus Tetrachlorkohlenstoff umkristallisiert werden. Die Ausbeute an 2,16-Dimethyl-3,15-dioxa-7,11-dioxo-5,13-dithia-4,14-dithioxoheptadecan beträgt 4,3 g (74%) und der Schmelzpunkt ist 98—100° C.

1,3-Bis(isodithionyl)-propan (s. Formel VIII mit R = H, X = S und E = $-(CH_2)_3-$)

In 3 ml konzentrierter Schwefelsäure werden in kleinen Portionen sehr langsam unter Eiskühlung 1,4 g (3,5 mMol) 2,16-Dimethyl-3,15-dioxa-7,11-dioxo-5,13-dithia-4,14-dithioxoheptadecan eingetragen. Man läßt noch 1 Stunde bei 0° C nachrühren und gießt dann das dunkelbraune Gemisch in Eiswasser ein. Es entsteht eine farblose Emulsion, die mit Ether ausgeschüttelt wird. Die Etherphase wird über Natriumsulfat getrocknet, eingeengt, und der Rückstand aus Cyclohexan umkristallisiert. Die Ausbeute beträgt 913 mg 1,3-Bis(isodithionyl)-propan (93%) und der Schmelzpunkt des farblosen Produktes ist 63—64° C.

1,3-Bis(isotrithionyl)-propan (s. Formel IX mit R = H, X = S und E = $-(CH_2)_3-$)

In 50 ml Xylol werden 1,3 g (4,7 mMol) 1,3-Bis(isodithionyl)-propan und 1 g Phosphorpentasulfid ($P_4S_{10}$) 2,5 Stunden am Rückfluß gekocht. Nach dem Abkühlen wird das Lösungsmittel abgegossen und eingeengt. Der Rückstand wird mehrmals mit Toluol ausgekocht und die Toluolphase ebenfalls eingeengt. Die vereinigten rotgelben Rückstände werden über eine Kieselgelsäure mit Methylenchlorid filtriert. Man erhält eine gelbe Fraktion, die eingeengt und nochmals über eine kurze Kieselgelsäule (Länge 15 cm, Ø 5 cm) gegeben wird. Hierbei ist das Laufmittel ein Gemisch aus Chloroform und Tetrachlorkohlenstoff im Verhältnis 7 : 3. Das Produkt kristallisiert auf der Säure aus und läuft sehr langsam. Nach der chromatographischen Abtrennung wird es aus Toluol umkristallisiert. Ausbeute an 1,3-Bis(isotrithionyl)-propan: 750 mg (52%). Der Schmelzpunkt beträgt 101—102° C.

1,3-Bis(2′-methylthio-1′,3′-dithiol-4′-yl)-propan (s. Formel X mit R = H, X = S und E = $-(CH_2)_3-$)

Eine Suspension von 1,23 g (4 mMol) des 1,3-Bis(isotrithionyl)-propans in 11 ml Nitromethan und 4 ml Methyljodid wird 2,5 Stunden am Rückfluß gekocht. Nach circa 30 Minuten fällt ein gelber Niederschlag aus. Man läßt abkühlen, fügt weitere 2 ml Methyljodid hinzu und läßt über Nacht bei Raumtemperatur nachrühren. Danach wird das Lösungsmittel mit Stickstoff abgeblasen und der orangegelbe Rückstand an der Ölpumpe getrocknet. Das so erhaltene Bissulfoniumjodid wird ohne weitere Reinigung (Rohausbeute: 2,36 g (100%)) wie folgt hydriert:

2,36 g = 4 mMol des rohen Bis-sulfoniumjodids werden in 21,5 ml absolutem Tetrahydrofuran und 21,5 ml absolutem Methanol suspendiert und auf −78° C abgekühlt. In 3 Portionen werden 1,5 g (40 mMol) Natriumborhydrid zugegeben und 3 Stunden bei −78° C nachgerührt. Man läßt innerhalb von 1,5 Stunden auf −20° C ansteigen, wobei das Reaktionsgemisch stark aufschäumt. Die gelbe Reaktionsmischung wird in 200 ml Ether gegossen und mit 200 ml Wasser gewaschen. Es bleibt eine gelbe Substanz zwischen den beiden Phasen ungelöst. Die Wasserphase wird zweimal mit 100 ml

Ether extrahiert. Die vereinigten Etherphasen werden zweimal mit 100 ml Wasser und einmal mit 100 ml einer gesättigten Natriumchlorid-Lösung gewaschen, über Natriumsulfat getrocknet und filtriert. Dann wird die etherische Lösung eingeengt und der stark gelbe Rückstand unter einer Stickstoffatmosphäre mit Methylenchlorid als Laufmittel über eine Kieselgelsäule (Länge 30 cm, Ø 1 cm) filtriert. Das Filtrat wird eingeengt und das Produkt bleibt in Form eines gelben empfindlichen Öls zurück. Die Ausbeute beträgt 1,044 g (77%).

Bis-dithioliumion (s. Formel XI mit R = H, E = $-(CH_2)_3-$ und X = S)

Unter Stickstoff werden 1,044 g (3,06 mMol) 1,3-Bis(2′-methylthio-1′,3′-dithiol-4′-yl)-propan in 21 ml Essigsäureanhydrid vorgelegt und auf 0° C abgekühlt. Dann addiert man tropfenweise innerhalb von 15 Minuten 2,91 ml Fluorborsäure. Man erhält eine braune Lösung, die noch 40 Minuten bei 0°C nachgerührt wird. In dieser Zeit fällt wenig hellbrauner Niederschlag aus. Es werden 200 ml absoluter Ether zugegeben, wobei ein dunkelbraunes Produkt ausfällt. Der Ether wird nach kurzem Durchrühren abpipettiert und der Niederschlag noch viermal mit je 100 ml Ether auf die gleiche Art und Weise gewaschen. Der restliche Ether wird mit Stickstoff abgeblasen und das Produkt an der Ölpumpe getrocknet. Der rotbraune Niederschlag wird ohne weitere Reinigung in der nächsten Stufe eingesetzt.

[3,3]-Tetrathiafulvalenophan

Unter einer Stickstoffatmosphäre gibt man bei Raumtemperatur langsam tropfenweise eine Lösung des rohen Bis-dithioliumions in 90 ml absolutem Acetonitril zu einer Lösung von 3,5 ml Triethylamin in 35 ml absolutem Acetonitril. Man läßt 40 Minuten nachrühren und engt dann am Rotationsverdampfer ein. Nach dem Trocknen an der Ölpumpe wird der rotbraune Rückstand mehrfach mit Schwefelkohlenstoff digeriert. Die Schwefelkohlenstoff-Lösung wird eingeengt und man erhält einen Rückstand in einer Rohausbeute von 130 mg (9%). Das NMR-Spektrum zeigt ein Hauptprodukt und möglicherweise Isomere. Zur weiteren Reinigung wird das [3,3]-Tetrathiafulvalenophan aus Schwefelkohlenstoff umkristallisiert.

Beispiel 3

Herstellung von [3]-Tetrathiafulvaleno[3]paracyclophan (s. Formel IV)

p-Phenylen-$\gamma,\gamma$-dibuttersäurechlorid

2,5 g (10 mMol) p-Phenylen-$\gamma,\gamma$-dibuttersäure werden in 34 ml thionylchlorid 2 Stunden am Rückfluß gekocht. Die Lösung färbt sich während der Reaktion dunkelbraun. Nach dem Abkühlen wird das Thionylchlorid abdestilliert und das zurückbleibende Öl wird mit 30 ml Petrolether versetzt. Das Säurechlorid löst sich, während Zersetzungsprodukte in Form eines teerartigen Rückstandes nicht gelöst werden. Die Lösung wird mit Aktivkohle geklärt, abfiltriert und eingeengt. Es entstehen leicht braune Kristalle, welche mit wenig Cyclohexan versetzt und dann abgesaugt werden. Man erhält farblose Kristalle mit einem Schmelzpunkt von 49—55° C. Die Ausbeute an p-Phenylen-$\gamma,\gamma$-dibuttersäurechlorid beträgt 2,38 g (83%).

1,4-Bis-(5′-diazo-4′-oxopentyl)-benzol

Hinter einer Schutzscheibe werden 600 ml (ca. 0,5 Mol) Diazomethan-Lösung in Ether in einem Erlenmeyerkolben mit Magnetrührer vorgelegt. Unter Eiskühlung werden 12 g (42 mMol) p-Phenyl-$\gamma,\gamma$-dibuttersäurechlorid gelöst in 200 ml Ether langsam zugetropft. Es ist eine Gasentwicklung zu beobachten. Es wird noch 3 Stunden nachgerührt und dann über Nacht stehen gelassen. Die gelbe Lösung wird abfiltriert und der Ether sowie das überschüssige Diazomethan mit Preßluft abgeblasen. Der feste gelbe Rückstand wird sofort in der nächsten Reaktion eingesetzt.

1,4-Bis-(5′-chloro-4′-oxopentyl)-benzol

Zu einem Gemisch aus 117 ml 5m-Natriumchlorid-Lösung und 117 ml 2N-Salzsäure-Lösung werden unter starkem Rühren ca. 42 mMol des in der vorhergehenden Reaktion erhaltenen 1,4-Bis-(5′-diazo-4′-oxopentyl)-benzols gelöst in 190 ml Methanol langsam getropft. Es tritt eine Trübung auf und eine Gasentwicklung ist zu beobachten. Es wird noch 30 Minuten nachgerührt und das ausgefallene gelbe

Rohprodukt (18,3 g) abgesaugt. Man chromatographiert mit Chloroform als Laufmittel über eine Kieselgelsäure (Länge 30 cm, ∅ = 7 cm) und erhält farblose Kristalle, die aus Isopropanol umkristallisiert werden. Der Schmelzpunkt des in 51%iger Ausbeute (6,7 g) erhaltenen 1,4-Bis-(5'-chloro-4'-oxopentyl)-benzols beträgt 70 bis 71° C.

1,4-Bis-(9'-methyl-8'-oxa-4'-oxo-6'-thia-7'-thioxo-decanyl)-benzol

( s. Formel VII mit X = S, R = H und E = $-(CH_2)_3-$ 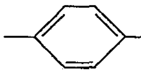 $-(CH_2)_3-$ )

6 g (18 mMol) 1,4-Bis-(5'-chloro-4'-oxopentyl)-benzol werden in 150 ml Aceton vorgelegt und 6,26 g (36 mMol) Kaliumisopropylxanthogenat werden zugegeben. Die Suspension wird 30 Minuten am Rükfluß gekocht, wobei ein farbloser Brei entsteht. Danach wird das Aceton abdestilliert und der Rückstand mit 100 ml Wasser versetzt. Man erhält eine Emulsion, die mit Ether extrahiert wird. Die Etherphase wird mit Wasser gewaschen und dann über Natriumsulfat getrocknet. Nach dem Entfernen des Lösungsmittels bleibt ein hellgelber Rückstand übrig, der im Kühlschrank kristallisiert. Das Produkt wird mit Methylenchlorid über Kieselgel (Länge 30 cm, ∅ 3 cm) chromatographiert und liegt danach in Form farbloser Kristalle und einer Ausbeute von 8,6 g (89%) vor. Der Schmelzpunkt beträgt 63—64° C.

1,4-Bis-(3'-isodithionylpropyl)-benzol

( s. Formel VIII mit R = H, X = S und E = $-(CH_2)_3-$ 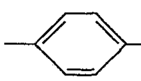 $-(CH_2)_3-$ )

In 8 ml eines Gemisches aus Ether-Chloroform (1 : 1) werden 0,5 g (1 mMol) 1,4-Bis-(9'-methyl-8'-oxa-4'-oxo-6'-thia-7'-thioxo-decanyl)-benzol gelöst. Unter Eiskühlung wird sehr langsam 0,75 ml Perchlorsäure eingetropft. Nachdem 30 Minuten nachgerührt wurde, wird in Eiswasser eingegossen, die organische Phase abgetrennt und die Wasserphase zweimal mit Chloroform extrahiert. Die organischen Phasen werden über Natriumsulfat getrocknet und dann eingeengt. Das resultierende Öl wird über eine Kieselgelsäule (Länge 30 cm, ∅ 1 cm) mit Methylenchlorid als Laufmittel filtriert. Man erhält 140 mg 1,4-Bis-(3'-isodithionylpropyl)-benzol in Form eines hellbraunen Öls. Da sich die Verbindung leicht zersetzt, wird sie umgehend wie folgt umgesetzt:

1,4-Bis-(3'-isotrithionylpropyl-benzol

( s. Formel IX mit R = H, X = S und E = $-(CH_2)_3-$ 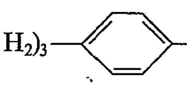 $-(CH_2)_3-$ )

380 mg (0,96 Mol) des rohen 1,4-Bis-(3'-isodithonylpropyl)-benzols werden in 15 ml Xylol vorgelegt und 500 mg Phosphorpentasulfid ($P_4S_{10}$) zugegeben. Es wird 2,5 Stunden bei einer Badtemperatur von 140°C am Rückfluß gekocht. Nach dem Abkühlen wird abfiltriert und der rotbraune Rückstand mit Toluol gewaschen. Die organische Phase wird eingeengt und über eine Kieselgelsäule (Länge 30 cm, ∅ 1 cm) mit einem Gemisch aus Tetrachlorkohlenstoff-Chloroform (7 : 3) chromatographiert. Man erhält das 1,4-Bis-(3'-isotrithionylpropyl)-benzol in Form gelber Kristalle. Ausbeute: 84 mg (27%) Fp: 99—100°C.

Die Gesamtausbeute über die letzten beiden Stufen beträgt 5 bis 10% und ist unabhängig von der Reinheit des 1,4-Bis-(3'-isodithionylpropyl)-benzols.

1,4-Bis-3'-(2"-methylthio-1",3"-dithiol-4"-yl)-proyplbenzol

( s. Formel X mit R = H, X = S und E = $-(CH_2)_3-$ 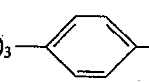 $-(CH_2)_3-$ )

1 g (2,34 mMol) 1,4-Bis-(3'-isotrithionylpropyl)-benzol werden in 6,25 ml destilliertem Nitromethan und 2,5 ml destilliertem Methyljodid 2,5 Stunden am Rückfluß gekocht (Badtemperatur 70° C). Man läßt die rotbraune Lösung abkühlen und fügt 1,2 ml Methyljodid hinzu. Es fällt ein gelbbrauner Niederschlag aus. Die Reaktionsmischung wird über Nacht nachgerührt. Danach wird das Lösungsmittel mit Stickstoff abgeblasen und der gelbbraune kristalline Rückstand an der Ölpumpe getrocknet. Die Rohausbeute beträgt 1,66 g (100%). Das so erhaltene Bissulfoniumjodid wird ohne weitere Säuberung wie folgt hydriert:

In 13 ml absolutem Tetrahydrofuran und 13 ml absolutem Methanol werden 1,66 (2,34 mMol) des rohen Bissulfoniumjodids suspendiert und auf $-78°$C abgekühlt. Man gibt in 3 Portionen 890 mg (23,4 mMol) Natriumtetrahydridoborat hinzu und rührt 3 Stunden bei $-78°$C. Dann läßt man innerhalb von 3 Stunden die Temperatur von $-78°$C auf $-20°$C ansteigen, wobei in der trüben, orangen Lösung eine starke Gasentwicklung zu beobachten ist. Das Reaktionsgemisch wird in Ether eingegossen und mit Wasser gewaschen. Die Wasserphase wird zweimal mit Ether extrahiert und dann die vereinigten Etherphasen zweimal mit 100 ml Wasser sowie einmal mit 100 ml gesättigter Natriumchlorid-Lösung gewaschen. Die organische Phase wird über Natriumsulfat getrocknet und dann eingeengt. Man erhält ein braunes Öl, welches über eine Kieselgelsäule (Länge 30 cm, ∅ 1 cm) mit Methylenchlorid chromatographiert wird. Nach dem Einengen erhält man ein leicht gelbes Öl in einer Ausbeute von 978 mg (91%). Die Ausbeute bezieht sich auf die beiden letzten Stufen.

Bis-dithioliumion

(s. Formel XI mit R = H, X = S und E = $—(CH_2)_3—$ 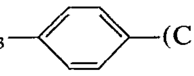 $—(CH_2)_3—$)

Unter Stickstoff werden 860 mg (1,89 mMol) 1,4-Bis-3′-(2″-methylthio-1″,3″-dithiol-4″-yl)-propylbenzol in 13 ml Essigsäureanhydrid vorgelegt und auf 0° C abgekühlt. Dann gibt man innerhalb einer halben Stunde 1,8 ml Fluorborsäure tropfenweise hinzu. Man erhält eine hellbraune Lösung, die noch 40 Minuten bei 0° C nachgerührt wird. In dieser Zeit fällt ein hellbrauner Niederschlag aus. Es werden 100 ml absoluter Ether hinzugegeben, womit man noch weiteren Niederschlag ausfällt. Man läßt gut durchrühren und pipettiert die etherische Lösung nach dem Absitzen des Niederschlages ab. Auf diese Weise wird noch viermal mit je 100 ml absolutem Ether gewaschen. Der restliche Ether wird dann mit Stickstoff abgeblasen und der Niederschlag an der Ölpumpe getrocknet. Das hellbraune Produkt wird ohne weitere Reinigung in der nächsten Stufe eingesetzt.

[3]-Tetrathiafulvaleno[3]-paracyclophan

Zu einer Lösung von 2,2 ml destilliertem Triethylamin in 22 ml absolutem Acetonitril tropft man bei Raumtemperatur unter einer Stickstoffatmosphäre langsam eine Lösung von 1,02 g (1,89 mMol) des Bis-dithioliumions aus der vorhergehenden Reaktion in 60 ml absolutem Acetonitril. Die Zugabe erfolgt am besten mit einer Spritze im Zeitraum von einer halben Stunde. Nach der Zugabe läßt man noch 40 Minuten nachrühren und engt dann am Rotationsverdampfer ein. Der Rückstand wird an der Ölpumpe getrocknet. Man hat ein rotbraunes festes Produkt, das hauptsächlich aus Polymeren besteht. Man addiert Ether in Portionen von je 30 ml und digeriert den Rückstand so lange, bis sich die Etherphase nicht mehr gelb färbt. Dazu werden ca. 300 ml Ether benötigt. Die vereinigten Ether-Lösungen werden über eine Florisilsäule filtriert (Länge 30 cm, ∅ 1 cm). Die Säule färbt sich sogleich grün. Die gelbe Etherfraktion wird eingeengt, wobei eine gelbe Substanz zurückbleibt, welche sofort kristallisiert.

Das NMR-Spektrum zeigt das cis- und trans-Isomer des [3]-Tetrathiafulvaleno[3]paracyclophans im Verhältnis von etwa 1 : 1. Die Ausbeute des Isomerengemisches beträgt 195 mg (29%). Durch mehrmaliges Umkristallisieren aus Cyclohexan konnte das trans-[3]Tetrathiafulvaleno[3]-paracyclophan isoliert werden. Fp.: 180—182° C.

Beispiel 4

Herstellung von [4]-Tetrathiafulvaleno[4]paracyclophan (s. Formel IV):

p-Phenylen-$\delta$,$\delta$-divaleriansäurechlorid

In 70 ml frisch destilliertes Thionylchlorid werden 6,95 g (25 mMol) p-Phenylen-$\delta$,$\delta$-divaleriansäure eingetragen. Es wird bei einer Badtemperatur von 90° C 2 Stunden am Rückfluß gekocht und danach das überschüssige Thionylchlorid abrotiert. Es bleibt ein dunkelbraunes Öl zurück, das mit Petrolether (40/60° C) in der Kälte verrührt wird bis sich ein schwarzes teerartiges Produkt am Rande absetzt (ca. 500 ml Petrolether). Die Petrolether-Lösung wird mit Aktivkohle versetzt und dann filtriert. Nach dem Einengen am Rotationsverdampfer bleibt ein leicht braunes Produkt zurück, welches im Kühlschrank kristallisiert. Da die Kristalle sehr schnell an der Luft zerfließen, wurde auf eine Charakterisierung verzichtet und das Rohprodukt, das in einer Ausbeute von 7,8 g (100%) erhalten wurde, direkt in der folgenden Reaktion eingesetzt.

1,4-Bis-(6′-diazo-5′-oxohexyl)-benzol

Die Reaktion ist hinter einer Schutzscheibe durchzuführen. 300 ml etherische Diazomethanlösung (ca. 0,25 Mol) werden in einem Erlenmeyerkolben mit Magnetrührer vorgelegt. Unter Eiskühlung wird langsam eine Lösung von 7,6 g (26,8 mMol) p-Phenylen-$\delta,\delta$-divaleriansäurechlorid in 100 ml Ether eingetropft. Hierbei ist eine Gasentwicklung festzustellen. Man läßt 24 Stunden stehen und bläst dann den Ether und das überschüssige Diazomethan mit Preßluft ab. Der gelbe feste Rückstand wird sofort in der nächsten Reaktionsstufe eingesetzt.

1,4-Bis-(6′-chlor-5′-oxohexyl)-benzol

Eine Lösung von etwa 27 mMol 1,4-Bis-(6′-diazo-5′-oxohexyl)-benzol (hergestellt aus 7,6 g p-Phenylen-$\delta,\delta$-divaleriansäurechlorid) in 70 ml Methanol wird eingetropft in ein Gemisch aus 58 ml 5m wäßriger Natriumchlorid-Lösung und 58 ml 2n wäßriger Salzsäure-Lösung. Beim Eintropfen muß die Vorlage gekühlt werden und stark gerührt werden. Nach dem Eintropfen und nachdem die Stickstoffentwicklung beendet ist, läßt man noch 4 Stunden bei Raumtemperatur nachrühren. Die Lösung wird mit 200 ml Wasser versetzt und mit Chloroform extrahiert. Die vereinigten Chloroformphasen werden mit 200 ml Wasser gewaschen und die schwach gelbe Lösung über Natriumsulfat getrocknet. Nach dem Abfiltrieren engt man am Rotationsverdampfer ein. Den gelben, festen Rückstand chromatographiert man mit Chloroform als Laufmittel über eine Kieselgelsäule (Länge 50 cm, Ø 5 cm). Das Bischlorketon läuft vorne. Nach dem Einengen der Fraktionen erhält man das farblose 1,4-Bis-(6′-chloro-5′-oxohexyl)-benzol, das aus Cyclohexan umkristallisiert wird. Der Schmelzpunkt, der in 53%iger Ausbeute (4,9 g) erhaltenen Substanz beträgt 86—87° C.

1,4-Bis-(10′-methyl-9′-oxa-5′-oxo-7′-thia-8′-thioxo-undecanyl)-benzol

(s. Formel VII mit X = S, R = H und E = 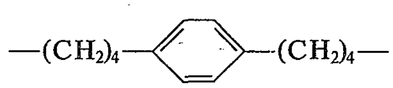 )

In eine Lösung von 4,5 g (13,1 mMol) 1,4-Bis-(6′-chloro-5′-oxohexyl)-benzol in 150 ml Aceton gibt man 5,0 g (28,8 mMol) Kaliumisopropylxanthat und kocht 30 Minuten am Rückfluß, wobei ein farbloser Niederschlag ausfällt. Die Suspension wird am Rotationsverdampfer eingeengt und der Rückstand mit 200 ml Wasser versetzt. Man extrahiert mit Chloroform und trocknet die organische Phase über Natriumsulfat. Nach dem Einengen am Rotationsverdampfer bleiben 12 g eines milchigen Öls zurück. Das Produkt wird mit Methylenchlorid als Laufmittel über eine Kieselgelsäule (Länge 50 cm, Ø 5 cm) chromatographiert. Man erhält farblose Kristalle, die aus Cyclohexan umkristallisiert werden. Die Ausbeute an 1,4-Bis-(10′-methyl-9′-oxa-5′-oxo-7′-thia-8′-thioxo-undecanyl)-benzol beträgt 5,3 g (75%). Fp = 114—115°C.

1,4-Bis-(4′-isodithionylbutyl)-benzol

(s. Formel VIII mit R = H, X = S und E = 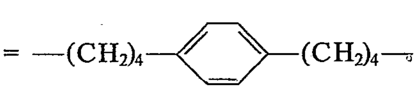 )

In einem Gemisch bestehend aus 4 ml Ether und 4 ml Methylenchlorid werden 0,54 g (1 mMol) 1,4-Bis-(10′-methyl-9′-oxa-5′-oxo-7′-thia-8′-thioxo-undecanyl)-benzol gelöst. In diese Lösung, die mit einem Eisbad gekühlt wird, tropft man sehr langsam (ca. 10 Minuten) 0,75 ml Perchlorsäure. Dann wird 1,5 Stunden am Rückfluß gekocht, wobei die Ölbadtemperatur 40—45° C beträgt. Nach dem Abkühlen gießt man in Eiswasser und läßt die Mischung 30 Minuten rühren. Danach werden die beiden Phasen getrennt und die wäßrige Phase noch einmal mit Methylenchlorid gewaschen. Die vereinigten organischen Phasen werden über Natriumsulfat getrocknet und nach dem Abfiltrieren bei 20° C Badtemperatur am Rotationsverdampfer eingeengt. Das zurückbleibende farblose Öl wird über eine Kieselgelsäule (Länge 30 cm, Ø 1 cm) chromatographiert. Als Laufmittel benutzt man ein Gemisch aus Tetrachlorkohlenstoff und Chloroform (1 : 1). Man erhält 1,4-Bis-(4′-isodithionylbutyl)-benzol als halbfestes, farbloses Produkt in einer Ausbeute von 276—330 mg (65—78%), das ohne weitere Reinigung in der folgenden Reaktion eingesetzt werden kann. Zur spektroskopischen Charakterisierung wurde eine Probe auf einer präparativen Dünnschichtplatte (Kieselgel) mit Tetrachlorkohlenstoff und Chloroform (1 : 1) als Laufmittel getrennt. Man erhält farblose Kristalle, die mit Ether verrieben und dann abgesaugt werden. Der Schmelzpunkt beträgt 86—88° C.

1,4-Bis-(4'-isotrithionylbutyl)-benzol

(s. Formel IX mit R = H, X = S und E = $-(CH_2)_4-$ 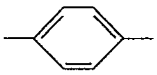 $-(CH_2)_4-$)

In 30 ml Xylol werden 828 mg (1,96 mMol) 1,4-Bis-(4'-isodithionylbutyl)-benzol gelöst und dann 1,74 g (3,92 mMol) Phosphorpentasulfid ($P_4S_{10}$) zugegeben. Bei einer Ölbadtemperatur von 140—150° C wird 2,5 Stunden am Rückfluß erhitzt. Die Reaktionsmischung färbt sich rotbraun, dann setzt dich ein rotbrauner Niederschlag am Rand ab und die Lösung ist jetzt stark gelb gefärbt.

Nach dem Abkühlen wird abfiltriert, der Rückstand gut mit warmem Toluol gewaschen und die vereinigten organischen Lösungen eingeengt. Es bleibt ein stark gelb gefärbtes Öl zurück, das mit Methylenchlorid über eine Florisilsäule (Länge 30 cm, ∅ 1 cm) filtriert wird. Nach dem Einengen erhält man ein gelbes, kristallines Produkt. Es wird mit einem Gemisch aus Tetrachlorkohlenstoff und Chloroform (7 : 3) über Florisil (Länge der Säule 30 cm, ∅ 1 cm) chromatographiert. Man erhält 1,4-Bis-(4'-isotrithionylbutyl)-benzol in einer Ausbeute von 431—530 mg (48—59%). Durch Umkristallisieren aus wenig Toluol erhält man gelbe Kristalle vom Schmelzpunkt 104—105° C.

1,4-Bis-[4'-(2"-methylthio-1",3"-dithiol-4"-yl)-butyl]-benzol

(s. Formel X mit R = H, X = S und E = $-(CH_2)_4-$ 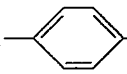 $-(CH_2)_4-$)

Eine Lösung von 837 mg (1,84 mMol) 1,4-Bis-(4'-isotrithionylbutyl)-benzol in 5 ml destilliertem Nitromethan und 2 ml destilliertem Methyljodid wird 2,5 Stunden zum Rückfluß erhitzt (Ölbadtemperatur ca. 70° C). Dann läßt man abkühlen, fügt 1 ml dest. Methyljodid hinzu und läßt 19 Stunden rühren. Während dieser Zeit ist ein Niederschlag ausgefallen und man erhält einen sehr viskosen Brei. Das Dünnschichtchromatogramm (Kieselgel—Methylenchlorid) zeigt keine Ausgangsverbindung mehr. Da das so erhaltene Bis-sulfoniumjodid sich unter Luft- und Feuchtigkeitseinfluß zersetzt, wird das Lösungsmittel mit Stickstoff abgeblasen, der gelbe Niederschlag an der Ölpumpe getrocknet und ohne Säuberung in der folgenden Reaktion eingesetzt. Rohausbeute des Bis-sulfoniumjodids: 1,36 g (100%).

Das rohe Bis-sulfoniumjodid (1,36 g, 1,84 mMol) wird in 10 ml absolutem Tetrahydrofuran und 10 ml absolutem Methanol suspendiert und auf −78° C gekühlt. Man addiert 700 mg (18,4 mMol) Natriumtetrahydridoborat in 3 Portionen und läßt 3 Stunden bei −78° C rühren. In einem Zeitraum von 1,5 Stunden erwärmt man die Reaktionsmischung von −78° C auf −20° C, gießt dann in Ether und wäscht mit Wasser. Die wäßrige Phase wird zweimal mit je 50 ml Ether gewaschen. Die vereinigten etherischen Lösungen werden zweimal mit je 100 ml Wassser und einmal mit 100 ml einer gesättigten Natriumchlorid-Lösung gewaschen und dann über Natriumsulfat getrocknet. Nach Abrotieren des Lösungsmittels erhält man ein rotbraunes Öl, das über Kieselgel (Länge 30 cm, ∅ 1 cm) mit Methylenchlorid chromatographiert wird. Es resultiert ein gelbes Öl in einer Ausbeute von 852 mg (95%), das NMR-spektroskopisch rein ist.

Bis-dithioliumion

(s. Formel XI mit R = H, X = S und E = $-(CH_2)_4-$ 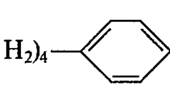 $-(CH_2)_4-$)

852 mg (1,75 mMol) 1,4-Bis-[4'-(2"-methylthio-1",3"-dithiol-4"-yl)-butyl]-benzol werden unter Stickstoff in 12 ml Essigsäureanhydrid emulgiert und auf 0° C gekühlt. Dann addiert man tropfenweise 1,7 ml einer 48%igen wäßrigen Fluorborsäure-Lösung in einem Zeitraum von 10 Minuten. Man erhält eine braune Lösung, die noch 40 Minuten bei 0° C gerührt wird. In dieser Zeit fällt ein farbloser Niederschlag aus. Danach gibt man 40 ml absoluten Ether hinzu und fällt auf diese Weise weiteres Produkt aus. Der Ether wird abpipettiert und der Niederschlag noch viermal gewaschen, indem man jeweils 40 ml absoluten Ether hinzugibt, die Reaktionsmischung durchrührt und nach dem Absitzen des Niederschlages den Ether mit einer Pipette entfernt. Nach dem letzten Waschen wird der restliche Ether mit Stickstoff abgeblasen und das rohe Bis-dithioliumion an der Ölpumpe getrocknet. Der leicht braune Niederschlag wird ohne weitere Reinigung in der nächsten Reaktion eingesetzt.

[4]-Tetrathiafulvaleno[4]-paracyclophan

In eine Lösung von 2 ml absolutem Triethylamin in 20 ml absolutem Acetonitril tropft man bei Raumtemperatur langsam (innerhalb von 35 Minuten) eine Lösung des rohen Bis-dithioliumions (992 mg, 1,75 mMol) in 55 ml absolutem Acetonitril unter einer Stikstoffatmosphäre. Man läßt weitere 40 Minuten nachrühren und entfernt dann das Lösungsmittel am Rotationsverdampfer. Nach dem Trocknen an der Ölpumpe extrahiert man den Rückstand mit mehreren Portionen Ether (so lange, bis sich die etherische Phase nicht mehr gelb färbt) und filtriert die etherischen Lösungen durch eine Florisilsäule (Länge 30 cm, Ø 1 cm). Nach Entfernen des Lösungsmittels erhält man braun-gelbe Kristalle, die erneut in ca 200 ml absolutem Ether gelöst und dann über eine Kieselgelsäule (Länge 30 cm, Ø 1 cm) filtriert werden. Nach dem Abrotieren des Lösungsmittels und Trocknen an der Ölpumpe erhält man das [4]-Tetrathiafulvaleno[4]-paracyclophan in form gelber Kristalle. Die Ausbeute beträgt 428 mg (63%) und das Produkt ist NMR-spektroskopisch rein.

Zur Charakterisierung wurde die Verbindung aus Cyclohexan umkristallisiert. Fp = 157—158° C.

**Patentansprüche**

1. Cyclisch substituierte Fulvalenophane der Formel

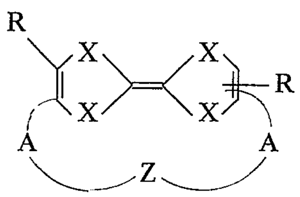

(I)

in der X S, Se oder Te, R H oder niederes Alkyl mit 1—5 C-Atomen, A einen $-(Ch_2)_n$-Rest, in dem n für 2, 3, 4 oder 5 steht und ein oder mehrere $-CH_2$-Glieder durch die Gruppen $-S-$, $-SO_2-$, $-O-$, $-NH-$, $-NR^1-$, $-CO-$, $-O-CO-$, $-O-CO-O-$, $-NR^1-CO-$, in denen $R^1$ für einen Alkylrest mit 1 bis 5 C-Atomen steht, ersetzt sein können, und Z einen Rest der Formel

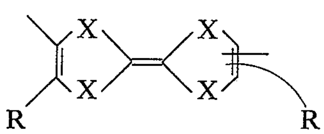

einen p-Phenylrest, der durch Halogenatome, Nitrogruppen oder Cyangruppen substituiert sein kann, oder einen Chinodimethanrest der Formel

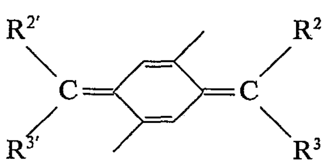

in dem die Reste $R^2$ und $R^3$ Halogenatome, Carbonestergruppen oder Cyangruppen bedeuten und $R^3$ außerdem für ein Wasserstoffatom stehen kann und der Chinodimethanring durch Halogenatome, Nitrogruppen oder Cyanogruppen substituiert sein kann, bedeuten.

2. Tetrathiafulvalenophane der Formel

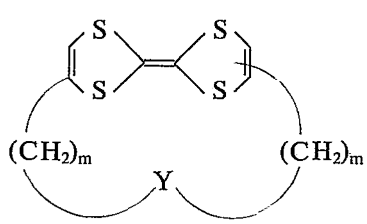

(II)

in der m die Zahl 2, 3, 4 oder 5 und Y einen der Reste

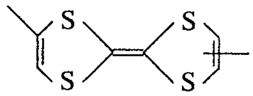

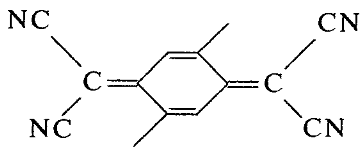

oder einen p-Phenylenring, der Cyanogruppen, Nitrogruppen oder Halogenatome enthalten kann, bedeuten.

3. Tetrathiafulvalenophane der Formel

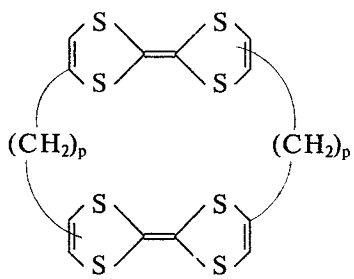

(III)

in der p für 2, 3 oder 4 steht.

4. Tetrathiafulvalenoparacyclophane der Formel

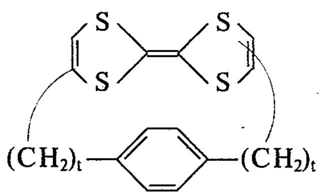

(IV)

in der t für 3, 4 oder 5 steht.

5. Verfahren zur Herstellung der cyclisch substituierten Fulvalenophane gemäß Anspruch 1, dadurch gekennzeichnet, daß man eine Verbindung der Formel

$$Hal—(R)HC—OC—E—CO—CH(R)—Hal \qquad (V)$$

in der Hal Chlor oder Brom und E einen $-(CH_2)_n-$Rest oder einen Rest der Formel $-A-Z-A-$ bedeuten

a) durch Umsetzung mit Verbindungen der Formel

$$KX—CX—OCH(CH_3)_2 \qquad (VI)$$

in eine Verbindung der Formel

$$[(CH_3)_2CH—O—CX—X—CH(R)—CO]_2E \qquad (VII)$$

überführt,

b) die Verbindung der Formel VII durch Behandlung mit Säuren zu einer Verbindung der Formel

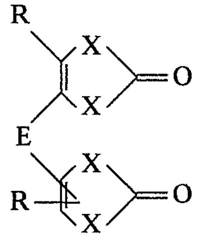

(VIII)

cyclisiert,

c) die Verbindung der Formel VIII durch Behandlung mit Phosphor-(V)-sulfid zu der Verbindung der Formel

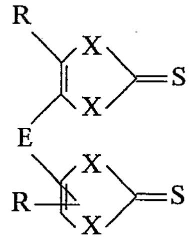

(IX)

umsetzt,

d) die Verbindung der Formel IX durch Methylierung und Reduktion in die Verbindung der Formel

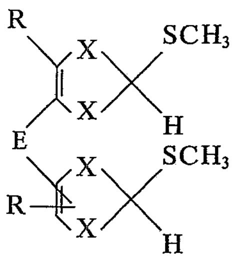

(X)

überführt,

e) die Verbindung der Formel X durch Behandlung mit Fluorborsäure in das Fluoborat der Formel

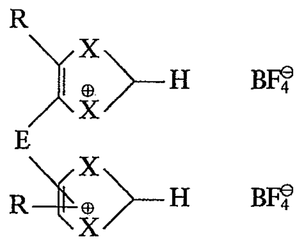

(XI)

überführt und

f) das Fluoborat der Formel XI mit tertiären Aminen behandelt.

6. Verwendung der Verbindungen gemäß Anspruch 1, für die Herstellung von organischen elektrischen Halbleitern oder elektrischen Leitern, dadurch gekennzeichnet, daß man sie als solche einsetzt oder mit Verbindungen der Formel

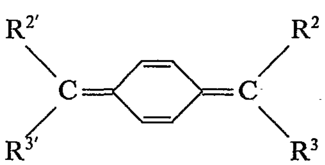

(XIII)

in der die Reste $R^2$ und $R^3$ Halogenatome, Carbonsäureestergruppen oder Cyangruppen bedeuten und $R^3$ außerdem für ein Wasserstoffatom stehen kann, und der Chinodimethanring durch Halogenatome, Nitrogruppen oder Cyanogruppen substituiert sein kann, oder mit substituierten Benzolen behandelt.

## Claims

1. A cyclically substituted fulvalenophane of the formula

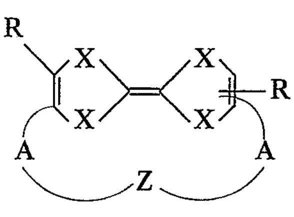

(I)

where X is S, Se or Te, R is H or lower alkyl of 1 to 5 carbon atoms, A is $-(CH_2)_n-$, where n is 2, 3, 4 or 5 and one or more of the $-CH_2-$ members may be replaced by $-S-$, $-SO_2-$, $-O-$, $-NH-$, $-NR^1-$, $-CO-$, $-O-CO-$, $-O-CO-O-$ or $-NR^1-CO-$, $R^1$ being alkyl of 1 to 5 carbon atoms, and Z is a radical of the formula

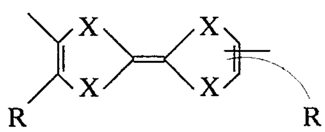

p-phenylene, which may substituted by halogen, nitro or cyano, or a quinodimethane radical of the formula

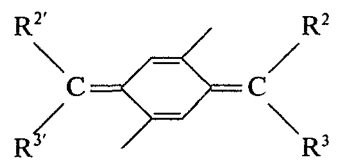

where $R^2$ and $R^3$ are halogen, carboxylic acid ester groups or cyano and $R^3$ may also be hydrogen, and the quinodimethane ring may be substitued by halogen, nitro or cyano.

2. A tetrathiafulvalenophane of the formula

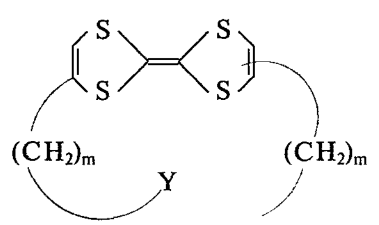

(II)

where m is 2, 3, 4 or 5 and Y is

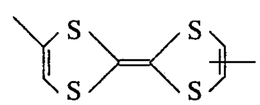

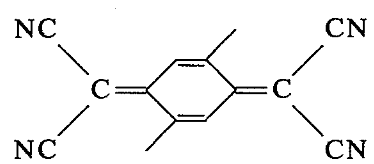

or p-phenylene which may contain cyano, nitro or halogen substituents.

3. A tetrathiafulvalenophane of the formula

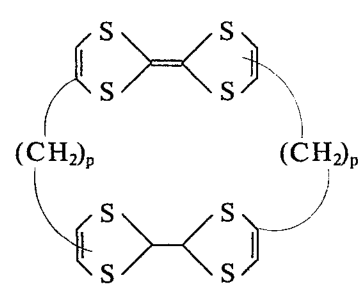

(III)

where p is 2, 3 or 4.

4. A tetrathiafulvalenoparacyclophane of the formula

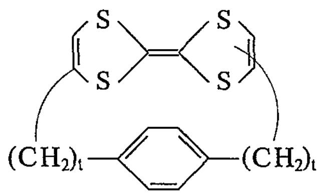

(IV)

where t is 3, 4 or 5.

5. A process for the production of a cyclically substituted fulvalenophane as claimed in claim 1, characterized in that a compound of the formula

$$Hal{-}(R)HC{-}OC{-}E{-}CO{-}CH(R){-}Hal \qquad (V)$$

where Hal is chlorine or bromine and E is $-(CH_2)_n-$ or $-A-Z-A-$,

a) is converted, by reaction with a compound of the formula

$$KX{-}CX{-}OCH(CH_3)_2 \qquad (VI)$$

into a compound of the formula

$$(CH_3)_2CH{-}O{-}CX{-}X{-}CH(R){-}CO_2E \qquad (VII)$$

b) the compound of the formula VII is cyclized by treatment with an acid, to give a compound of the formula

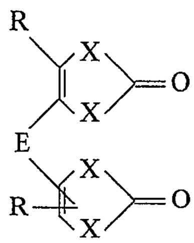

(VIII)

c) the compound of the formula VIII is treated with phosphorus (V) sulfide to give the compound of the formula

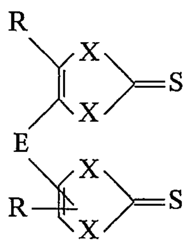

(IX)

d) the compound of the formula IX is converted to the compound of the formula

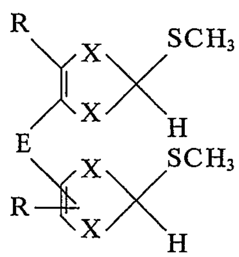

(X)

by methylation and reduction,

e) the compound of the formula X is converted to the tetrafluoborate of the formula

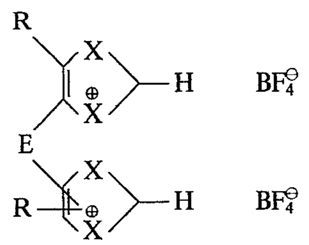

(XI)

by treatment with fluoboric acid and

f) the fluoborate of the formula XI is treated with a tertiary amine.

6. Use of the compounds as claimed in claim 1 for the production of organic electric semiconductors or electric conductors, characterized in that they are used as such or are treated with compounds of the formula

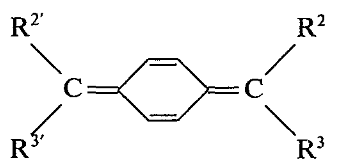

(XIII)

where $R^2$ and $R^3$ are halogen, carboxylic acid ester groups or cyano groups and $R^3$ may also be hydrogen, and the quinodimethane ring may be substitued by halogen, nitro or cyano, or with substituted benzenes.

**Revendications**

1. Fulvalènophanes cycliquement substitués de formule

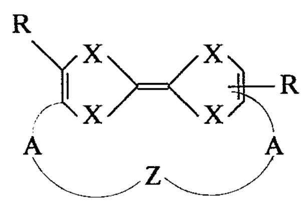

(I)

dans laquelle X représente S, Se ou Te; R représente H ou un alcoyle inférieur à 1–5 atomes de carbone; A représente un radical $-(CH_2)_n$ dans lequel n est mis pour 2, 3, 4 ou 5 et l'un ou plusieurs des termes $-CH_2$ peut être remplacé par les groupes $-S-$, $-SO_2-$, $-O-$, $-NH-$, $-NR^1-$, $-CO-$, $-O-CO-$, $-O-CO-O-$, $-NR^1-CO-$, $R^1$ étant mis pour un radical alcoyle à 1–5 atomes de carbone; et Z représente un radical de formule

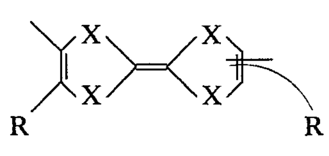

un radical p-phénylène, qui peut être substitué par des atomes d'halogènes, des groupes nitro ou des groupes cyano, ou un radical quinodiméthane de formule

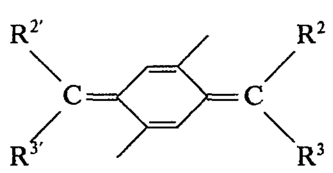

dans laquelle les radicaux $R^2$ et $R^3$ représentent des atomes d'halogènes, des groupes esters d'acides

carboxyliques ou des groupes cyano et, en outre, $R^3$ peut être mis pour un atome d'hydrogène, et le noyau quinodiméthane peut être substitué par des atomes d'halogènes, des groupes nitro ou des groupes cyano.

2. Tétrathiafulvalènophanes de formule

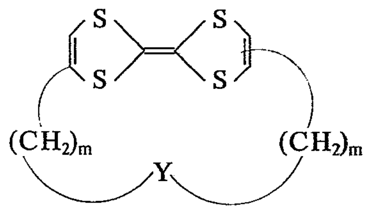

(II)

dans laquelle m est mis pour l'un des nombres 2, 3, 4 ou 5 et Y représente l'un des radicaux

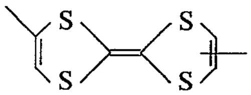

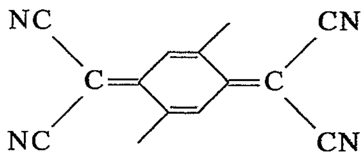

ou un noyau p-phénylène, qui peut contenir des groupes cyano, des groupes nitro ou des atomes d'halogènes.

3. Tétrathiafulvalènophanes de formule

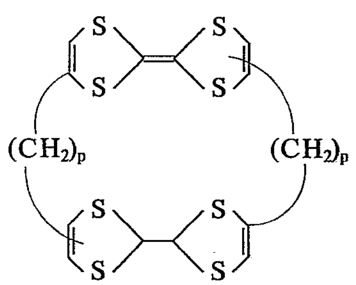

(III)

dans laquelle p est mis pour 2, 3 ou 4.

4. Tétrathiafulvalènoparacyclophanes de formule

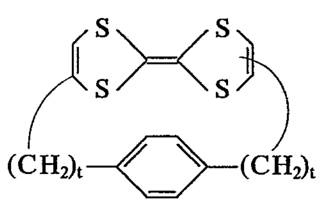

(IV)

dans laquelle t est mis pour 3, 4 ou 5.

5. Procédé pour la préparation des fulvalènophanes cycliquement substitués selon la revendication 1, caractérisé en ce que:

a) l'on transforme un composé de formule

$$Hal—(R)HC—OC—E—CO—CH(R)—Hal \qquad (V)$$

dans laquelle Hal représente un chlore ou un brome et E un radical $-(CH_2)_n-$ ou un radical de formule $-A-Z-A-$, par réaction avec des composés de formule

$$KX—CX—OCH(CH_3)_2 \qquad (VI)$$

en un composé de formule

$$(CH_3)_2CH—O—CX—X—CH(R)—CO_2E \qquad (VII)$$

b) l'on cyclise le composé de formule VII par traitement par des acides pour obtenir un composé de formule

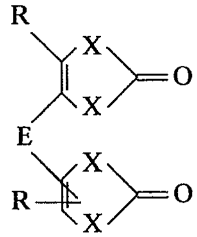

(VIII)

c) l'on convertit le composé de formule VIII, par traitement par le pentasulfure de phosphore, en composé de formule

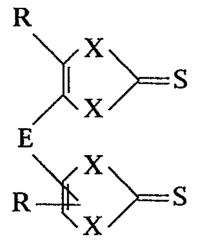

(IX)

d) l'on transforme le composé de formule IX, par méthylation et réduction, en composé de formule

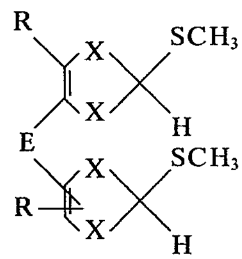

(X)

e) l'on transforme le composé de formule X, par traitement par l'acide fluoborique, en fluoborate de formule

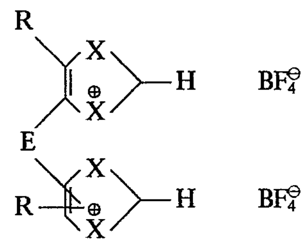

(XI)

f) et l'on traite le fluoborate de formule XI par des amines tertiaires.

6. Utilisation des composés selon la revendication 1 pour la fabrication des semiconducteurs électriques organiques ou de conducteurs électriques, caractérisée en ce qu'on les met en oeuvre tels quels ou on les traite par des composés de formule

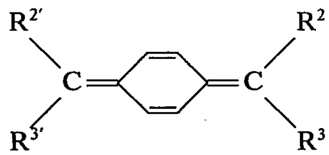

(XIII)

dans laquelle les radicaux $R^2$ et $R^3$ représentent des atomes d'halogènes, des groupes esters d'acides carboxyliques ou des groupes cyano et, en outre, $R^2$ peut être mis pour un atome d'hydrogène et le noyau quinodiméthane peut être substitué par des atomes d'halogènes, des groupes nitro ou des groupes cyano, ou on les traite par des benzènes substitués.